# FASCICULE DE BREVET EUROPEEN

(11) **EP 1 557 124 B1**
(45) Date de publication et mention de la délivrance du brevet: **28.02.2007**
(21) Numéro de dépôt: 05356012.4
(22) Date de dépôt: 19.01.2005
(51) Int. Cl.: A61B 5/15

(54) **Connecteur de sécurité à aiguille**
Nadel-Sicherheits-Verbinder
Needle safety connector

(30) Priorité: 22.01.2004 FR 0400681
(43) Date de publication de la demande: 27.07.2005
(73) Titulaire: P2A Medical, 73410 Albens (FR)
(72) Inventeur: Orlu, Alain, 74540 Viuz la Chiesaz (FR); Commaret, Pierre-André, 1279 Bogis-Bossey (CH)
(74) Mandataire: Poncet, Jean-François

(56) Documents cités:
- EP-A- 0 908 193
- GB-A- 1 303 211
- US-A- 4 784 650
- US-A- 5 084 034
- US-A1- 2001 041 866

## Description

La présente invention concerne les connecteurs de sécurité permettant de connecter un récipient de prélèvement à une canalisation de conduction de fluide. L'invention concerne en particulier un tel connecteur destiné au prélèvement sanguin dans les applications du domaine de la santé.

Lors de prélèvements sanguins, notamment pour les opérations de dons de sang, on doit systématiquement effectuer une analyse de ce sang en prélevant une petite quantité de sang dans un récipient de prélèvement. Pour cela, une canalisation de conduction de fluide doit être raccordée de manière amovible et étanche à un récipient de prélèvement, dans des conditions d'hygiène absolue, en nécessitant un minimum de mouvement de l'opérateur pour la connexion du récipient de prélèvement à la canalisation et pour sa déconnexion.

Simultanément, il faut assurer la protection de l'opérateur contre tous risques de blessure et de contamination par le sang prélevé.

Pour cela, on a déjà imaginé un connecteur de sécurité à aiguille tel que décrit dans le document US 4,784,650, comprenant une aiguille creuse de passage de fluide, un manchon support d'aiguille, et un cylindre protecteur. Le manchon support d'aiguille porte l'aiguille en la raccordant de manière étanche à la canalisation de conduction de fluide à laquelle il est fixé. Une portion dépassante d'aiguille prolonge axialement le manchon support d'aiguille à l'opposé de la canalisation de conduction de fluide. Le cylindre protecteur définit un espace intérieur contenant la partie dépassante d'aiguille, avec une extrémité distale ouverte pour l'introduction du récipient de prélèvement à connecter par l'aiguille, et avec une extrémité proximale fermée de raccordement au manchon support d'aiguille.

En pratique, il est nécessaire de réaliser sous forme d'éléments séparés le manchon support d'aiguille et le cylindre protecteur, de façon à utiliser des matériaux différents susceptibles de présenter les propriétés satisfaisantes pour l'encliquetage d'une part et la tenue ferme de l'aiguille dans le manchon support d'aiguille d'autre part.

Pour cela, les dispositifs connus réalisent le connecteur de sécurité en deux parties essentielles à assembler l'une à l'autre, à savoir un manchon support d'aiguille et un cylindre protecteur, avec des moyens de raccordement assurant un raccordement par encliquetage du cylindre protecteur sur le manchon support d'aiguille.

Les moyens de raccordement comportent un cylindre proximal à grand diamètre à ouverture proximale, suivi d'un cylindre distal à plus petit diamètre dont le fond communique avec l'espace intérieur du cylindre protecteur par un passage d'aiguille. Des moyens de retenue sont prévus pour retenir axialement le manchon support d'aiguille engagé dans le cylindre proximal et dans le cylindre distal.

Dans les dispositifs connus, les moyens de retenue comportent des trous d'encliquetage prévus dans la paroi latérale du cylindre distal, et dans lesquels s'engagent des excroissances du manchon support d'aiguille.

Les connecteurs de sécurité à aiguille connus présentent des inconvénients qui affectent la sécurité d'utilisation. Notamment, on constate un défaut de résistance à l'arrachement de l'aiguille ; il peut se produire parfois un écoulement de gouttes de sang hors du flacon de prélèvement lors des mouvements de retrait du flacon, et ces gouttes de sang risquent de s'écouler par les trous d'encliquetage du cylindre proximal ; on constate également un risque non négligeable de déformation permanente du cylindre distal par enfoncement lors de l'engagement du manchon support d'aiguille dans le cylindre distal et le cylindre proximal ; une telle déformation permanente augmente encore les risques de défaut d'étanchéité, et d'écoulement de sang hors du cylindre protecteur ; également, l'encliquetage du cylindre protecteur sur le manchon support d'aiguille est difficile à contrôler, car le déplacement axial d'encliquetage est faible.

Le problème proposé par la présente invention est de concevoir une nouvelle structure de connecteur de sécurité à aiguille qui soit facile à assembler sur le manchon support d'aiguille par simple encliquetage, qui soit étanche, qui puisse être fermé pour sécurité après usage, et qui retienne suffisamment l'aiguille pour empêcher tout déplacement sous l'action de la force axiale d'enfoncement du flacon de prélèvement.

Pour atteindre ces buts ainsi que d'autres, l'invention propose un connecteur de sécurité à aiguille, comprenant une aiguille creuse de passage de fluide, un manchon support d'aiguille, et un cylindre protecteur, le manchon support d'aiguille portant l'aiguille en la raccordant de manière étanche à une canalisation de conduction de fluide à laquelle il est fixé, une portion dépassante d'aiguille prolongeant axialement le manchon support d'aiguille à l'opposé de la canalisation de conduction de fluide, le cylindre protecteur ayant un espace intérieur contenant la partie dépassante d'aiguille, avec une extrémité distale ouverte pour l'introduction d'un récipient de prélèvement à connecter par l'aiguille, et avec une extrémité proximale fermée à moyens de raccordement au manchon support d'aiguille ; les moyens de raccordement comportent un cylindre proximal à grand diamètre à ouverture proximale, suivi d'un cylindre distal à plus petit diamètre dont le fond communique avec l'espace intérieur du cylindre protecteur par un passage d'aiguille, et comportent des moyens de retenue pour retenir axialement le manchon support d'aiguille engagé dans le cylindre proximal et dans le cylindre distal ; selon l'invention :
- les parois latérales du cylindre proximal et du cylindre distal sont étanches,
- le manchon support d'aiguille comprend une collerette d'appui, conformée pour pénétrer avec un jeu périphérique approprié dans le cylindre proximal et pour venir buter contre un épaulement intermédiaire reliant le cylindre proximal et le cylindre distal,
- le cylindre proximal comprend des moyens de languettes de verrouillage élastiquement flexibles, orientées au repos en direction oblique vers l'extrémité proximale depuis une zone intermédiaire du cylindre protecteur pour venir en appui contre la face distale de la collerette d'appui, puis, après fléchissement vers l'aval des moyens de languettes de verrouillage lors de la pénétration du manchon support d'aiguille, pour venir en appui contre la face proximale de la collerette d'appui en s'opposant ainsi au retrait du manchon support d'aiguille hors du cylindre protecteur.

Selon un mode de réalisation avantageux, les moyens de languettes de verrouillage comprennent deux languettes diamétralement opposées.

Pour assurer une bonne étanchéité de l'aiguille après retrait du flacon de prélèvement, un manchon d'obturation est engagé autour de la partie dépassante d'aiguille et traverse le passage d'aiguille prévu dans le fond du cylindre distal, en assurant l'étanchéité entre l'aiguille et le fond du cylindre distal. Simultanément, le manchon est rétractable par déformation élastique axiale lorsque le flacon est engagé autour de l'aiguille, et il se déploie à nouveau axialement autour de l'aiguille pour obturer son orifice distal lors du retrait du flacon de prélèvement.

En pratique, le manchon support d'aiguille comprend une collerette d'étanchéité, décalée en amont de la collerette d'appui, et venant en appui contre les moyens de languettes de verrouillage lorsque le manchon support d'aiguille est engagé dans le cylindre protecteur.

D'autres objets, caractéristiques et avantages de la présente invention ressortiront de la description suivante de modes de réalisation particuliers, faite en relation avec les figures jointes, parmi lesquelles :
- la figure 1 est une illustration schématique globale d'un système de prélèvement sanguin utilisant un connecteur de sécurité à aiguille selon la présente invention ;
- la figure 2 est une vue partielle de détail agrandie en coupe longitudinale du connecteur de sécurité à aiguille selon un mode de réalisation de l'invention, avant assemblage ;
- la figure 3 est une vue à plus grande échelle du connecteur de sécurité de la figure 1, à l'état assemblé, en coupe longitudinale selon le plan A-A de la figure 4 ; et
- la figure 4 est une vue en bout du connecteur de sécurité de la figure 3.

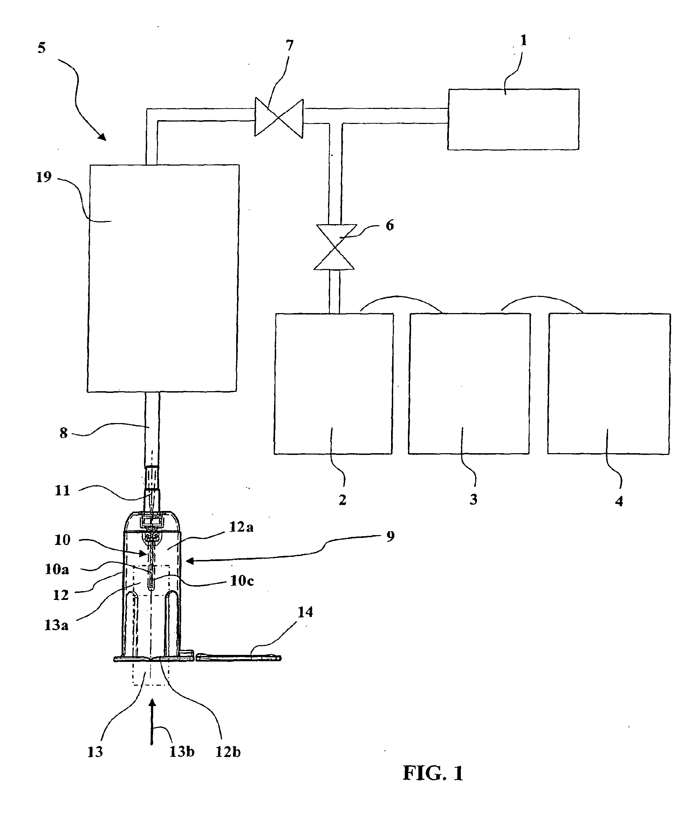

On considère tout d'abord la figure 1, qui illustre la structure générale d'un dispositif de prélèvement sanguin mettant en oeuvre un connecteur de sécurité selon la présente invention.

A partir d'une source de sang schématiquement illustrée par la référence 1, par exemple un cathéter engagé dans le système veineux d'un patient, des canalisations conduisent le sang soit à une série de poches 2, 3 et 4, soit à un ensemble de prélèvement de test 5, le choix de communication étant assuré par des vannes 6 et 7 pilotées par l'opérateur.

L'ensemble de prélèvement de test 5 comporte par exemple, en série dans une canalisation de conduction de fluide 8, une poche tampon 19. L'extrémité de la canalisation de conduction de fluide 8 comprend le connecteur de sécurité à aiguille 9 selon l'invention.

Le connecteur de sécurité à aiguille 9 comporte une aiguille creuse de passage de fluide 10, un manchon support d'aiguille 11 et un cylindre protecteur 12. Le manchon support d'aiguille 11 est fixé à l'extrémité de la canalisation de conduction de fluide 8, et porte l'aiguille 10 en la raccordant de manière étanche à la canalisation de conduction de fluide 8. L'aiguille 10 comporte une portion dépassante d'aiguille 10a qui prolonge axialement le manchon support d'aiguille 11 à l'opposé de la canalisation de conduction de fluide 8.

Le cylindre protecteur 12 définit un espace intérieur 12a dont la forme est adaptée pour contenir entièrement la partie dépassante d'aiguille 10a, de façon à éviter tout contact d'une main d'un opérateur avec la partie d'extrémité agressive 10c de l'aiguille 10. Par ailleurs, le cylindre protecteur 12 comporte une extrémité distale 12b ouverte pour l'introduction d'un récipient de prélèvement 13 à connecter à la canalisation de conduction de fluide 8. L'extrémité distale 12b peut être obturée par un clapet rabattable 14. De façon connue, le récipient de prélèvement 13 comporte un bouchon 13a en un matériau pouvant être percé par la partie dépassante d'aiguille 10a, et la forme du récipient de prélèvement 13 est adaptée pour être engagée dans l'espace intérieur 12a du cylindre protecteur 12. Par pression axiale 13b du récipient de prélèvement 13 en direction de l'aiguille 10, on provoque le percement du bouchon 13a par l'aiguille 10, qui met ainsi en communication l'intérieur du récipient de prélèvement 13 et la canalisation de conduction de fluide 8, pour l'introduction d'une quantité appropriée de sang dans le récipient de prélèvement 13. On peut ensuite retirer le récipient de prélèvement 13 par simple extraction axiale hors du cylindre protecteur 12 : le bouchon 13a se referme élastiquement sur lui-même pour assurer l'étanchéité du récipient de prélèvement 13.

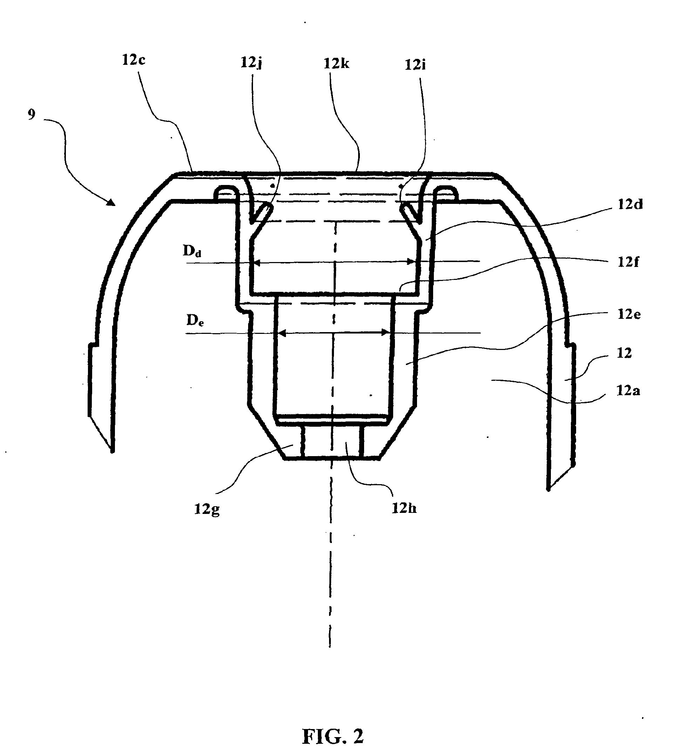

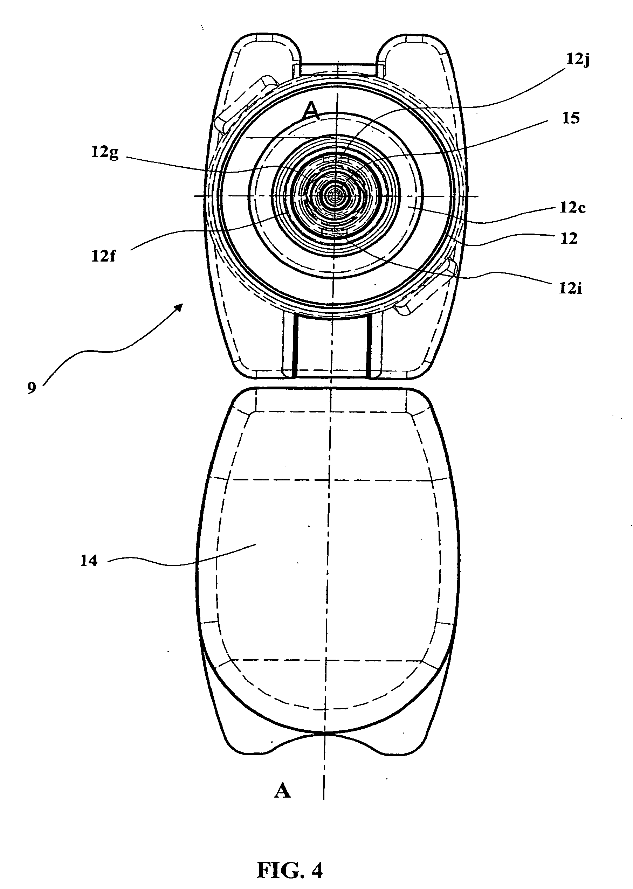

On considère maintenant la structure détaillée du connecteur de sécurité 9, telle qu'elle apparaît sur les figures 2 à 4.

Sur ces figures, on retrouve le manchon support d'aiguille 11, l'aiguille 10 et le cylindre protecteur 12.

Le cylindre protecteur 12 comporte une extrémité proximale 12c fermée et munie de moyens de raccordement au manchon support d'aiguille 11. Les moyens de raccordement sont adaptés pour permettre l'assemblage du cylindre protecteur 12 et du manchon support d'aiguille 11 par encliquetage lors d'un simple mouvement de translation axiale 11e l'un vers l'autre. Simultanément, le même mouvement de translation axiale 11 e l'un vers l'autre assure l'étanchéité de l'assemblage, tout écoulement de sang depuis l'espace intérieur 12a du cylindre protecteur 12 vers sa surface extérieure et vers le manchon support d'aiguille 11 étant rendu impossible par des moyens d'étanchéité.

Les moyens de raccordement du cylindre protecteur 12 comportent un cylindre proximal 12d ayant un diamètre intérieur D_{d} plus grand que le diamètre du manchon support d'aiguille 11. Le cylindre proximal 12d est suivi d'un cylindre distal 12e à diamètre intérieur De plus petit, avec un épaulement intermédiaire 12f qui relie le cylindre proximal 12d et le cylindre distal 12e.

Le cylindre distal 12e se termine par un fond 12g qui communique avec l'espace intérieur 12a du cylindre protecteur 12 par un passage d'aiguille 12h de faible diamètre.

Les parois latérales du cylindre proximal 12d et du cylindre distal 12e sont étanches, c'est-à-dire dépourvues de toute ouverture susceptible de permettre le passage d'un fluide tel que le sang.

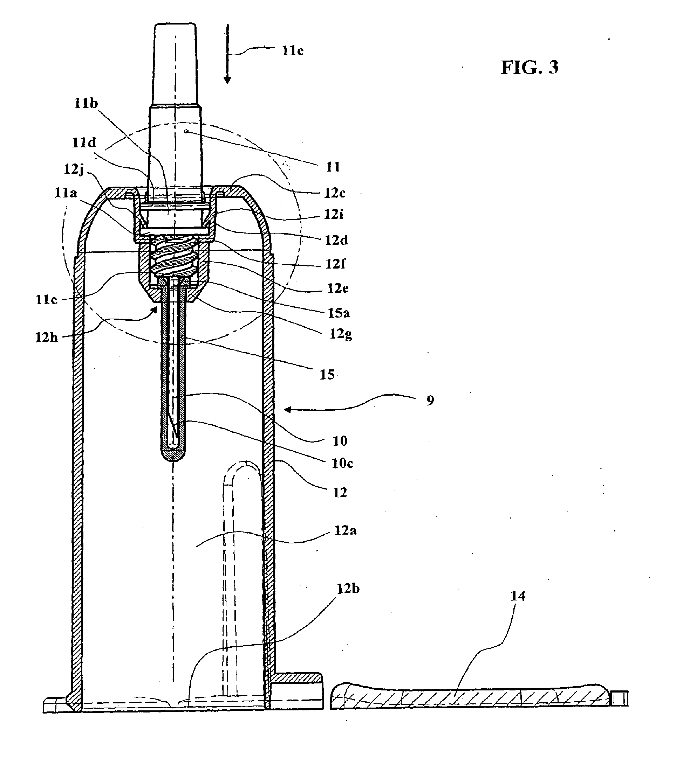

Le manchon support d'aiguille 11 comprend une collerette d'appui 11 a, conformée pour pénétrer dans le cylindre proximal 12d et pour venir buter contre l'épaulement intermédiaire 12f lorsque le manchon support d'aiguille 11 est engagé en position d'assemblage sur le cylindre protecteur 12 comme illustré sur la figure 3.

Le cylindre proximal 12d comprend des moyens de languettes de verrouillage élastiquement flexibles, par exemple les deux languettes diamétralement opposées 12i et 12j, coopérant avec la collerette d'appui 11 a pour réaliser les moyens d'encliquetage s'opposant à la séparation du manchon support d'aiguille 11 et du cylindre protecteur 12 lorsqu'ils sont en position assemblée.

Pour cela, les languettes de verrouillage 12i et 12j élastiquement flexibles sont orientées au repos en direction oblique vers l'extrémité proximale 12k du cylindre proximal 12d, comme on le voit sur la figure 2, depuis une zone intermédiaire du cylindre proximal 12d, et leurs extrémités libres sont situées l'une par rapport à l'autre à une distance inférieure au diamètre de la collerette d'appui 11a. De la sorte, lorsqu'on approche le manchon support d'aiguille 11 vers le cylindre protecteur 12, la collerette d'appui 11 a vient en appui sur les extrémités libres des languettes de verrouillage 12i et 12j, en les repoussant vers l'épaulement 12f. Les languettes de verrouillage 12i et 12j fléchissent alors pour prendre une orientation oblique inverse en direction de l'épaulement 12f, comme on le voit sur la figure 3.

Par le fait que l'on prévoit, entre la collerette d'appui 11 a et la paroi périphérique du cylindre proximal 12d, un jeu périphérique approprié, ou par le fait de l'élasticité du matériau constituant le cylindre proximal 12d qui peut s'écarter légèrement au passage de la collerette d'appui 11a entre les languettes de verrouillage 12i et 12j, la collerette d'appui 11a peut poursuivre sa course pour venir buter contre l'épaulement intermédiaire 12f, les languettes de verrouillage 12i et 12j venant alors en appui contre la face proximale de la collerette d'appui 11a comme on le voit sur la figure 3. Dans cette position, les languettes de verrouillage 12i et 12j sont également en appui sur la portion cylindrique 11 b adjacente du manchon support d'aiguille 11, cette portion cylindrique 11 b ayant un diamètre suffisant pour maintenir les languettes de verrouillage 12i et 12j en orientation oblique en direction de l'épaulement 12f. Les languettes de verrouillage 12i et 12j s'opposent alors au retrait du manchon support d'aiguille 11 hors du cylindre protecteur 12.

Dans la réalisation illustrée sur les figures 1, 3 et 4, un manchon d'obturation 15 est engagé autour de la partie dépassante d'aiguille 10a, et traverse le passage d'aiguille 12h dans le fond 12g du cylindre distal 12e, en assurant l'étanchéité entre l'aiguille 10 et le fond 12g du cylindre distal 12e. Simultanément, le manchon d'obturation 15 est en matériau élastiquement rétractable, et obture l'extrémité libre 10c de l'aiguille 10 en l'absence d'un flacon de prélèvement 13 engagé sur l'aiguille 10.

Dans la position d'assemblage illustrée sur la figure 3, une portion d'extrémité proximale 15a du manchon d'obturation 15 est comprimée axialement entre l'extrémité distale 11 c du manchon support d'aiguille 11 et le fond 12g du cylindre distal 12e. On améliore encore ainsi l'étanchéité.

Dans la réalisation illustrée sur les figures 1 et 3, le manchon support d'aiguille 11 comprend en outre une collerette d'étanchéité 11d, décalée en amont de la collerette d'appui 11a, et venant en appui contre les languettes de verrouillage 12i et 12j lorsque le manchon support d'aiguille 11 est engagé dans le cylindre protecteur 12.

Le cylindre protecteur 12 peut être avantageusement réalisé en une première matière plastique moulée telle que le polypropylène.

Simultanément, le manchon support d'aiguille 11 peut être en une seconde matière plastique moulée telle que le polycarbonate.

Le manchon d'obturation 15 est avantageusement en une mousse élastique à cellules fermées, ou en un élastomère élastiquement flexible tel que le latex.

La présente invention n'est pas limitée aux modes de réalisation qui ont été explicitement décrits, mais elle en inclut les diverses variantes et généralisations contenues dans le domaine des revendications ci-après.

## Revendications

1. - Connecteur de sécurité à aiguille (9) comprenant une aiguille (10) creuse de passage de fluide, un manchon support d'aiguille (11), et un cylindre protecteur (12), le manchon support d'aiguille (11) portant l'aiguille (10) en la raccordant de manière étanche à une canalisation de conduction de fluide (8) à laquelle il est fixé, une portion dépassante d'aiguille (10a) prolongeant axialement le manchon support d'aiguille (11) à l'opposé de la canalisation de conduction de fluide (8), le cylindre protecteur (12) ayant un espace intérieur (12a) contenant la partie dépassante d'aiguille (10a), avec une extrémité distale (12b) ouverte pour l'introduction d'un récipient de prélèvement (13) à connecter par l'aiguille (10), et avec une extrémité proximale (12c) fermée à moyens de raccordement au manchon support d'aiguille (11), les moyens de raccordement comportant un cylindre proximal (12d) à grand diamètre (D_{d}) à ouverture proximale, suivi d'un cylindre distal (12e) à plus petit diamètre (Dₑ) dont le fond (12g) communique avec l'espace intérieur (12a) du cylindre protecteur (12) par un passage d'aiguille (12h), et comportant des moyens de retenue pour retenir axialement le manchon support d'aiguille (11) engagé dans le cylindre proximal (12d) et dans le cylindre distal (12e),
**caractérisé en ce que :**
- les parois latérales du cylindre proximal (12d) et du cylindre distal (12e) sont étanches,
- le manchon support d'aiguille (11) comprend une collerette d'appui (11a), conformée pour pénétrer avec un jeu périphérique approprié dans le cylindre proximal (12d) et pour venir buter contre un épaulement intermédiaire (12f) reliant le cylindre proximal (12d) et le cylindre distal (12e),
- le cylindre proximal (12d) comprend des moyens de languettes de verrouillage élastiquement flexibles (12i, 12j), orientées au repos en direction oblique vers l'extrémité proximale depuis une zone intermédiaire du cylindre protecteur (12) pour venir en appui contre la face distale de la collerette d'appui (11a), puis, après fléchissement vers l'aval des moyens de languettes de verrouillage (12i, 12j) lors de la pénétration du manchon support d'aiguille (11), pour venir en appui contre la face proximale de la collerette d'appui (11a) en s'opposant ainsi au retrait du manchon support d'aiguille (11) hors du cylindre protecteur (12).

2. - Connecteur de sécurité selon la revendication 1, **caractérisé en ce que** les moyens de languettes de verrouillage comprennent deux languettes (12i, 12j) diamétralement opposées.

3. - Connecteur de sécurité selon l'une des revendications 1 ou 2, **caractérisé en ce qu**'un manchon d'obturation (15) est engagé autour de la partie dépassante d'aiguille (10a) et traverse le passage d'aiguille (12h) dans le fond (12g) du cylindre distal (12e) en assurant l'étanchéité entre l'aiguille (10) et le fond (12g) du cylindre distal (12e).

4. - Connecteur de sécurité selon l'une quelconque des revendications 1 à 3, **caractérisé en ce que** le manchon support d'aiguille (11) comprend une collerette d'étanchéité (11d), décalée en amont de la collerette d'appui (11a), et venant en appui contre les moyens de languettes de verrouillage (12i, 12j) lorsque le manchon support d'aiguille (11) est engagé dans le cylindre protecteur (12).

5. - Connecteur de sécurité selon l'une quelconque des revendications 1 à 4, **caractérisé en ce que** le cylindre protecteur (12) est en une première matière plastique moulée telle que le polypropylène.

6. - Connecteur de sécurité selon l'une quelconque des revendications 1 à 5, **caractérisé en ce que** le manchon support d'aiguille (11) est en une seconde matière plastique moulée telle que le polycarbonate.

7. - Connecteur de sécurité selon l'une quelconque des revendications 1 à 6, **caractérisé en ce que** le manchon d'obturation (15) est en une mousse élastique à cellules fermées ou en un élastomère élastiquement flexible tel que le latex.

## Claims

1. Safety needle connector (9) comprising a hollow needle (10) through which fluid can pass, a needle support sleeve (11), and a protective cylinder (12), the needle support sleeve (11) carrying the needle (10) and connecting it and sealing it to a fluid tube (8) to which it is fixed, a projecting needle portion (10a) axially extending the needle support sleeve (11) at the end opposite the fluid tube (8), the protective cylinder (12) having an interior space (12a) containing the projecting needle portion (10a), with an open distal end (12b) for inserting a sampling container (13) to be connected by the needle (10) and with a closed proximal end (12c) with connection means for connecting it to the needle support sleeve (11), the connection means comprising a proximal cylinder (12d) with a large diameter (D_{d}) and a proximal opening followed by a distal cylinder (12e) with a smaller diameter (Dₑ) whose bottom (12g) communicates with the interior space (12a) of the protective cylinder (12) via a needle passage (12h) and comprising retaining means for axially retaining the needle support sleeve (11) engaged in the proximal cylinder (12d) and in the distal cylinder (12e),
which safety needle connector is **characterized in that**:
- the lateral walls of the proximal cylinder (12d) and the distal cylinder (12e) are fluid-tight,
- the needle support sleeve (11) comprises a bearing flange (11a) conformed to penetrate with appropriate peripheral clearance into the proximal cylinder (12d) and to abut against an intermediate shoulder (12f) connecting the proximal cylinder (12d) and the distal cylinder (12e),
- the proximal cylinder (12d) comprises elastically flexible locking tongue means (12i, 12j) which when at rest, and starting from an intermediate region of the protective cylinder (12), are oriented in an oblique direction toward the proximal end to bear against the distal face of the bearing flange (11a), and then, following flexing in the downstream direction of the locking tongue means (12i, 12j) upon penetration of the needle support sleeve (11), to bear against the proximal face of the bearing flange (11a), thereby opposing withdrawal of the needle support sleeve (11) from the protective cylinder (12).

2. Safety connector according to claim 1, **characterized in that** the locking tongue means comprise two diametrically opposite tongues (12i, 12j).

3. Safety connector according to either claim 1 or claim 2, **characterized in that** a closure sleeve (15) is engaged around the projecting needle portion (10a) and passes through the needle passage (12h) in the bottom (12g) of the distal cylinder (12e) to provide a seal between the needle (10) and the bottom (12g) of the distal cylinder (12e).

4. Safety connector according to any one of claims 1 to 3, **characterized in that** the needle support sleeve (11) comprises a sealing flange (11d), offset in the upstream direction from the bearing flange (11a) and bearing against the locking tongue means (12i, 12j) when the needle support sleeve (11) is engaged in the protective cylinder (12).

5. Safety connector according to any one of claims 1 to 4, **characterized in that** the protective cylinder (12) is molded from a first plastic material such as polypropylene.

6. Safety connector according to any one of claims 1 to 5, **characterized in that** the needle support sleeve (11) is molded from a second plastic material such as polycarbonate.

7. Safety connector according to any one of claims 1 to 6, **characterized in that** the closure sleeve (15) is made from a closed cell resilient foam or an elastically flexible elastomer such as latex.

## Patentansprüche

1. Nadel-Sicherheitsverbinder (9) mit einer Hohlnadel (10) für den Durchlauf von Flüssigkeit, einer Nadelaufnahmemanschette (11) und einem Schutzzylinder (12), wobei die Nadelaufnahmemanschette (11) die Nadel (10) so hält, daß sie diese in abgedichteter Weise mit einer Flüssigkeitsleitung (8) verbindet, an der die Manschette befestigt ist, wobei ferner ein vorstehender Nadelabschnitt (10a) die Nadelaufnahmemanschette (11) auf der von der Flüssigkeitsleitung (8) abgewandten Seite in axialer Richtung verlängert und wobei der Schutzzylinder (12) einen Innenraum (12a) hat, der den vorstehenden Nadelabschnitt (10a) aufnimmt und der ein offenes distales Ende (12b) für die Einführung eines mit der Nadel (10) zu verbindenden Entnahmegefäßes (13) sowie ein proximales Ende (12c) aufweist, das über Mittel zur Verbindung mit der Nadelaufnahmemanschette (11) geschlossen ist, welche Verbindungsmittel einen proximalen Zylinder (12d) mit großem Durchmesser (D_{d}) und proximaler Öffnung haben, an den sich ein distaler Zylinder (12e) mit kleinerem Durchmesser (Dₑ) anschließt, dessen Boden (12g) über eine Nadeldurchtrittsöffnung (12h) mit dem Innenraum (12a) des Schutzzylinders (12) in Verbindung ist, wobei die Verbindungsmittel ferner Mittel zum axialen Festhalten der Nadelaufnahmemanschette (11) aufweisen, die in den proximalen Zylinder (12d) und in den distalen Zylinder (12e) eingreift,
**dadurch gekennzeichnet, daß**:
- die Seitenwände des proximalen Zylinders (12d) und des distalen Zylinders (12e) dicht sind,
- die Nadelaufnahmemanschette (11) eine Stützschulter (11a) aufweist, die so ausgebildet ist, daß sie mit einem geeigneten Umfangsspiel in den proximalen Zylinder (12d) eingreift und zur Anlage an einer mittleren Schulter (12f) kommt, die den proximalen Zylinder (12d) und den distalen Zylinder (12e) verbindet,
- der proximale Zylinder (12d) elastisch flexible Rastzungenmittel (12i, 12j) aufweist, die im Ruhezustand von einem mittleren Bereich des Schutzzylinders (12) ausgehend in schräge Richtung auf das proximale Ende ausgerichtet sind, um zur Anlage an der distalen Seite der Stützschulter (11a) zu kommen, worauf sie beim Eindringen der Nadelaufnahmemanschette (11) nach einer nach vorn gerichteten Durchbiegung zur Anlage an der proximalen Seite der Stützschulter (11a) kommen, so daß sie **dadurch** einem Austritt der Nadelaufnahmemanschette (11) aus dem Schutzzylinder (12) entgegenwirken.

2. Sicherheitsverbinder nach Anspruch 1, **dadurch gekennzeichnet, daß** die Rastzungenmittel zwei einander diametral gegenüberliegende Rastzungen (12i, 12j) haben.

3. Sicherheitsverbinder nach einem der Ansprüche 1 oder 2, **dadurch gekennzeichnet, daß** eine Verschlußhülse (15) auf den vorstehenden Nadelabschnitt (10a) aufgesetzt ist und durch die Durchtrittsöffnung (12h) für die Nadel im Boden (12g) des distalen Zylinders (12e) hindurchtritt, womit die Dichtwirkung zwischen der Nadel (10) und dem Boden (12g) des distalen Zylinders (12e) gewährleistet ist.

4. Sicherheitsverbinder nach einem der Ansprüche 1 bis 3, **dadurch gekennzeichnet, daß** die Nadelaufnahmemanschette (11) einen Abdichtkragen (11d) aufweist, der mit Abstand über der Stützschulter (11a) liegt und zur Anlage an den Rastzungenmitteln (12i, 12j) kommt, wenn die Nadelaufnahmemanschette (11) in den Schutzzylinder (12) eingesetzt ist.

5. Sicherheitsverbinder nach einem der Ansprüche 1 bis 4, **dadurch gekennzeichnet, daß** der Schutzzylinder (12) aus einem ersten formgepreßten Kunststoff wie beispielsweise Polypropylen besteht.

6. Sicherheitsverbinder nach einem der Ansprüche 1 bis 5, **dadurch gekennzeichnet, daß** die Nadelaufnahmemanschette (11) aus einem zweiten formgepreßten Kunststoff wie beispielsweise Polycarbonat besteht.

7. Sicherheitsverbinder nach einem der Ansprüche 1 bis 6, **dadurch gekennzeichnet, daß** die Verschlußhülse (15) aus einem elastischen Schaumstoff mit geschlossenen Zellen oder aus einem elastisch flexiblen Elastomer wie beispielsweise Latex besteht.
